# EUROPÄISCHE PATENTSCHRIFT

(11) **EP 1 658 262 B1**
(45) Veröffentlichungstag und Bekanntmachung des Hinweises auf die Patenterteilung: **20.02.2013**
(21) Anmeldenummer: 04764280.6
(22) Anmeldetag: 19.08.2004
(51) Int. Cl.: C07C 211/62, C07F 9/54, C07C 209/68, C07C 381/12

(54) **VERFAHREN ZUR HERSTELLUNG IONISCHER FLÜSSIGKEITEN, IONISCHER FESTSTOFFE ODER GEMISCHE DERSELBEN**
METHOD FOR PRODUCING IONIC LIQUIDS, IONIC SOLIDS OR MIXTURES THEREOF
PROCEDE POUR PRODUIRE DES LIQUIDES IONIQUES, DES SOLIDES IONIQUES OU LEURS MELANGES

(30) Priorität: 27.08.2003 AT 13512003
(43) Veröffentlichungstag der Anmeldung: 24.05.2006
(60) Teilanmeldung: 10011988.2
(73) Patentinhaber: proionic Production of Ionic Substances GmbH & Co KG, 8074 Grambach (AT)
(72) Erfinder: KALB, Roland, A-1020 Wien (AT)
(74) Vertreter: Vinazzer, Edith
(86) Internationale Anmeldenummer: PCT/EP2004/009296
(87) Internationale Veröffentlichungsnummer: WO 2005/021484

(56) Entgegenhaltungen:
- EP-A- 1 182 196
- WO-A-02/079212
- WO-A-03/022812
- WO-A-03/051894
- M. VISCONTINI ET AL.: "Synthese einiger Methylimino-diessigsäurederivate" HELV. CHIM. ACTA 35, 1952, Seiten 451-455, XP009044807
- E. ALCALDE ET AL.: "Novel Bis-betaines and Betaines within [1four]meta-Heterophane Frameworks" CHEM. EUR. J., Bd. 8, Nr. 2, 2002, Seiten 474-484, XP009044815
- J. HOWITZ ET AL.: "Ueber p-Oxy-chinolone und einige Halogenalkylate des ana-Brom-p-Oxychinolins" CHEM. BER. 38, 1905, Seite 888, XP009044883

## Beschreibung

Die Erfindung betrifft ein Verfahren zur Herstellung ionischer Flüssigkeiten, ionischer Feststoffe oder Gemische derselben.

Ionische Verbindungen - Verbindungen, welche ionische Flüssigkeiten oder ionische Feststoffe sind - weisen äußerst interessante Eigenschaften auf, wie beispielsweise einen nicht messbaren Dampfdruck, einen sehr großen Liquidusbereich, gute elektrische Leitfähigkeit und ungewöhnliche Solvatations-Eigenschaften. Diese Eigenschaften prädestinieren sie für den Einsatz in verschiedenen Bereichen technischer Anwendungen. So können sie beispielsweise als Lösungsmittel (bei organischer und anorganischer Synthese im Allgemeinen, bei der Übergangsmetallkatalyse, der Biokatalyse, der Phasentransfer-Katalyse, bei Mehrphasen-Reaktionen, in der Photochemie, in der Polymersynthese und der Nanotechnologie), als Extraktionsmittel (bei der flüssig-flüssig- und der flüssig-gasförmigen-Extraktion im Allgemeinen, der Entschwefelung von Rohöl, der Entfernung von Schwermetallen aus Abwässern, der Flüssigmembranextraktion), als Elektrolyte (in Batterien, Brennstoffzellen, Kondensatoren, Solarzellen, Sensoren, in der Elektrochromie, der Galvanotechnik, in der elektrochemischen Metallbearbeitung, in der elektrochemischen Synthese im Allgemeinen, bei der elektroorganischen Synthese, der Nanotechnologie), als Schmierstoffe, als Thermofluide, als Gele, als Reagenzien zur organischen Synthese, in der "Green Chemistry" (Ersatz für Volatile Organic Compounds), als Antistatika, in Spezialanwendungen der Analytik (Gaschromatographie, Massenspektroskopie, Kapillarzonenelektrophorese), als Flüssigkristalle, etc. eingesetzt werden. Diesbezüglich wird beispielsweise auf "Rogers, Robin D.; Seddon, Kenneth R. (Eds.); Ionic Liquids - Industrial Applications to Green Chemistry, ACS Symposium Series 818, 2002; ISBN 0841237891" und "Wasserscheid, Peter, Welton, Tom (Eds.); Ionic Liquids in Synthesis, Verlag Wiley-VCH 2003; ISBN 3527305157" verwiesen.

Die WO-A-03051894 offenbart ein Verfahren zur Herstellung ionischer Flüssigkeiten der Formel [Q⁺][A⁻] durch Umsetzung organischer Halide [Q⁺][X⁻] mit Broenstedt-Säuren. Als [Q⁺] kommen organische Katione in Frage, die quaternierte Stickstoff- oder Phosphoratome enthalten.

Die Optimierung der Eigenschaften für die jeweilige Anwendung kann in weiten Grenzen durch eine Variation der Struktur von Anion und Kation bzw. eine Variation ihrer Kombination erfolgen, was den ionischen Flüssigkeiten die Bezeichnung "Designer Solvents" (siehe beispielsweise Freemantle, M.; Chem. Eng. News, 78, 2000, 37) eingebracht hat. In diesem Sinne wurden viele unsystematische Einzelsynthesen für die Herstellung ionischer Verbindungen im Labor- und Technikumsmaßstab entwickelt. Dementsprechend sind die Kosten der Produktion dieser Verbindungen extrem hoch und die Umsetzbarkeit im Industriemaßstab kaum realisierbar.

Die Produktion ionischer Flüssigkeiten ist bis dato beinahe ausschließlich im Labor- bzw. Technikumsmaßstab möglich, wobei es keine flexible, allgemein anwendbare und effiziente Synthesemethode gibt und insbesondere für beinahe jedes gewünschte Anion ein spezielles Verfahren zur Anwendung kommt. Ausgangsverbindungen dieser Synthesen sind im Allgemeinen alkyl- oder arylsubstituierte organische Stickstoff-, Phosphor- oder Schwetelnucleophile (Amine, Phosphine, Sulfide, Heteroaromaten). Die derzeit bekannten Synthesemethoden werden am Beispiel der Synthese quatemärer Ammoniumverbindungen (siehe nachstehende Abbildung 1) erläutert:

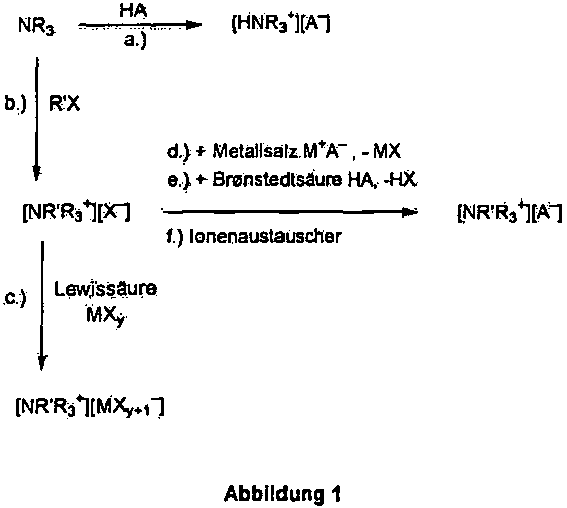

### a. Brønstedt-Säure/Basenreaktion:

Ein Amin R₃N reagiert mit einer Protonensäure HA unter Bildung des Salzes [R₃HN⁺[A⁻].

Diese Darstellung ionischer Flüssigkeiten ist die älteste bekannte (Walden, P., Bull. Acad. Imper. Sci. (St. Petersburg), 1914, 1800) und führt beispielsweise bei der Reaktion von Ethylamin mit Salpetersäure zur Bildung von Ethylammoniumnitrat mit einem Schmelzpunkt von Fₚ = 13°C. In diesem Fall ist das Kation nicht quatemiert sondern nur protoniert. Diese organischen Salze kommen für typische Anwendungsgebiete ionischer Flüssigkeiten nicht in Frage, weil sie thermisch und chemisch instabil sind. Sie werden hier nur der Vollständigkeit halber erwähnt und sind nicht Gegenstand der vorliegende Erfindung.

### b. Alkylierung bzw. Arylierung zu einem quaternären Salz:

Das Amin R₃N wird mit einem Alkylierungs- oder Arylierungsreagens R'X in einer nucleophilen Substitution unter Bildung eines quatemierten Ammoniumsalzes [NR'R₃⁺][X⁻] umgesetzt (Wilkes, J.S.; Zaworotko, M.J., J. Chem. Soc., Chem. Commun., 13, 1992, 965). Als typische Alkylierungsmittel kommen dabei Haloalkane wie z.B. 1-Chlorbutan, 1-Bromethan, Methyliodid oder Dialkylsulfate wie beispielsweise Dimethylsulfat oder Diethylsulfat zur Anwendung. Diese Alkylierungsmittel sind zwar reaktiv und bilden rasch das gewünschte Produkt, sie sind aber wie alle starken Alkylierungsreagenzien relativ toxisch, zum Teil cancerogen und im Falle der Haloalkane auch atmosphärenschädigend (stratosphärisches Ozon). Die gebildete ionische Verbindung [NR'R₃⁺][X⁻] kann bereits eine lonische Flüssigkeit sein (Fp < 100°C), dient aber in der Regel als Ausgangsmaterial der folgenden Reaktionen c - f. Sie ist im allgemeinen stark hygroskopisch.

### c. Reaktion mit einer Lewis-Säure:

Zur Darstellung von ionischen Flüssigkeiten auf der Basis von Halometallat-Anionen (siehe z.B. Wilkes, J.S.; Levisky, J.A.; Wilson, R.A.; Hussey, C.L., Inorg. Chem. 1982, 21, 1263) kann das quaternäre Ammoniumsalz [NR'R₃⁺][X⁻] direkt mit einer entsprechenden Lewis-Säure (Elektronenpaarakzeptor) MX_{y} zur gewünschten Verbindung [NR'R₃⁺][MX_{y+1}⁻] umgesetzt werden. Im Falle des Tetrachloroaluminat-Anions [AlCl₄]⁻ wird z.B. ein entsprechendes Ammoniumchlorid [NR'R₃⁺][Cl⁻] direkt mit Aluminiumchlorid AlCl₃ versetzt.

Auf Halometallat basierende ionische Flüssigkeiten sind extrem wasser- und sauerstoffempfindlich, sehr korrosiv und werden nur für ganz spezielle Zwecke eingesetzt (beispielsweise Olefin-Oligomerisierung, Friedel-Crafts Reaktionen). Bereits geringste Spuren Wasser führen zur Hydrolyse unter Bildung von HCl, HBr oder HI. Ihre Synthese gestaltet sich dementsprechend schwierig, nicht zuletzt auch aufgrund der Tatsache, dass das Ausgangsmaterial [NR'R₃⁺][X⁻] stark hygroskopisch ist. Sie werden hier nur der Vollständigkeit halber erwähnt und sind nicht Gegenstand der vorliegenden Erfindung.

### d. Anionenaustausch via Metathese:

Das durch Alkylierung bzw. Arylierung gebildete quaternäre Ammoniumsalz [NR'R₃⁺][X⁻] (siehe b.) trägt im Allgemeinen nicht das gewünschte Anion. Um das Halogenid- oder Sulfat-Anion [X⁻] gegen das gewünschte Anion [A⁻] auszutauschen, wird das Salz [NR'R₃⁺][X⁻] in einem trockenen, organischen Lösungsmittel (z.B. Aceton, Acetonitril, Methylenchlorid) mit einer stöchiometrischen Menge eines Metallsalzes (meist ein entsprechendes Alkali- oder Erdalkalisalz) [M⁺][A⁻] versetzt, welches das gewünschte Anion [A⁻] aufweist. Der Reaktionsansatz wird unter Ausschluss von Luftfeuchtigkeit typischerweise für einige Tage bis Wochen kräftig geführt, wobei die gewünschte ionische Flüssigkeit in dem gewählten Lösungsmittel löslich ist und das Anion [X⁻] als unlösliches, festes Metallsalz [M⁻][X⁻] ausfällt und durch Filtration entfernt wird. Neben der sehr langen Reaktionszeit und den großen Mengen von Abfall [M⁺][X⁻] ist bei diesem Verfahren von Nachteil, dass man trockene Lösungsmittel verwenden muss, um einen kompletten Umsatz zu erzielen; ansonsten löst sich das zu entfernende Salz [M⁺][X⁻] in größeren Mengen im Lösungsmittel, wobei diese Halogenidspuren den späteren Einsatz der ionischen Flüssigkeit limitieren (Halogenide sind beispielsweise Katalysatorgifte; Entsorgung durch Verbrennen ist problematisch).

Das wasserfreie Arbeiten ist insbesondere im Industriemaßstab problematisch, da die Vorstufe [NR'R₃⁺][X⁻] im Allgemeinen sehr hygroskopisch ist und der Produktionsprozess daher unter Ausschluss jeglicher Luftfeuchtigkeit erfolgen muss. Die genannten Nachteile könnten zwar durch den Einsatz von wässrigen Lösungsmittelsystemen und Silbersalzen [Ag⁺][A⁻] behoben werden (wobei die auch in Wasser sehr schwerlöslichen Silberhalogenide ausfallen), dies ist allerdings im Industriemaßstab aus ökonomischen Gründen völlig ausgeschlossen.

Ein kürzlich zum Patent angemeldetes einstufiges Produktionsverfahren (Wasserscheid, Peter.; Hilgers, Claus; Boesmann, Andreas; EP1182197 A1 (Solvent Innovation GmbH, Germany), 2002), welches die Reaktionen b und d vereint, kann zwar das Problem des Ausschlusses von Luftfeuchtigkeit zum Teil entschärfen (keine Isolation der hygroskopischen Zwischenstufe [NR'R₃⁺][X⁻] notwendig), allerdings bleiben die Reaktionszeiten nach wie vor äußerst lang, es fällt weiterhin Abfall an und die potentielle Gefahr der Verunreinigung des Endproduktes mit Halogenid-Ionen ist allgegenwärtig; darüber hinaus lassen sich Kondensationsprodukte und Verunreinigungen, die bei der Darstellung der hier nicht isolierten Zwischenstufe entstehen, nur nachträglich unter erheblich größerem Aufwand aus dem fertigen Produkt entfernen.

Allen Anionenaustauschverfahren via Metathese ist aber insbesondere gemeinsam, dass sie nicht allgemein anwendbar sind und nur für ganz bestimmte Kombinationen aus Kationen und Anionen zufriedenstellend funktionieren. Nur wenn die gewünschte ionische Flüssigkeit im Lösungsmittel ausreichend löslich ist (kann oft problematisch sein, zum Teil große Mengen Lösungsmittel nötig) und/oder das eingesetzte Metallsalz [M⁺][A⁻] im Lösungsmittel wesentlich besser löslich ist als das zu entfernende, kann mit Erfolg gerechnet werden. Des weiteren kann das ausgefällte Salz [M⁺][X⁻] zum Teil in der ionischen Flüssigkeit selbst löslich sein.

### e. Anionenaustausch via Br⌀nstedt-Säure:

Eine weitere Variante des Anionenaustausches stellt die Reaktion der ionischen Vorstufe [NR'R₃⁺][X⁻] mit einer Br⌀nstedtsäure HA dar. In diesem Fall muss die zugehörige freie Säure des gewünschten Anions stärker sein als die entsprechende Säure HX, sodass auch diese Reaktion nur für wenige Anionen [A⁻] in Frage kommt. Ist HX - wie im Falle der Halogenwasserstoffsäuren - eine flüchtige Säure, kann diese verhältnismäßig leicht im Vakuum entfernt werden (ionische Flüssigkeiten besitzen im Allgemeinen keinen messbaren Dampfdruck); ist die ionische Flüssigkeit hydrophob, kann die Säure HX mit Wasser extraktiv entfernt werden. In allen anderen Fällen gestaltet sich die Isolation des Produktes schwierig.

### f. Anionenaustausch an einem lonentauscherharz:

Der Austausch des Ions [X⁻] gegen das gewünschte Ion [A⁻] kann auch in einem allgemein bekannten Verfahren an einem herkömmlichen Anionentauscherharz erfolgen; aus ökonomischer Sicht ist dieses Verfahren im Industriemaßstab aber kaum anwendbar, da die maximale Beladung eines Harzes sehr beschränkt ist.

Der Erfindung liegt die Aufgabe zu Grunde, ein Verfahren zur Herstellung ionischer Flüssigkeiten oder ionischer Feststoffe zur Verfügung zu stellen, welches es gestattet, eine sehr breite Palette dieser Substanzen herstellen zu können. Die ionischen Flüssigkeiten oder ionischen Feststoffe sollen in industrierelevanten Mengen "ontime" und kundenspezifisch herstell- und lieferbar sein und beim Hersteller nur einen geringen Lageraufwand erfordern.

Gelöst wird die gestellte Aufgabe erfindungsgemäß dadurch, dass die ionischen Flüssigkeiten oder ionischen Feststoffe modular aus kationischen Synthesemodulen [Q⁺]ₙ[Yⁿ⁻] und aus anionischen Synthesemodulen hergestellt werden, wobei
[Q⁺] ein quaterniertes Ammonium- [R¹R²R³R⁴N⁺] Phosphonium- [R¹R²R³R⁴P⁺] oder Sulfonium-[R¹R²R³S⁺] Kation oder ein analoger quaternierter Stickstoff-, Phosphor- oder Schwefel-Heteroaromat ist,
wobei die Reste R¹R²,R³,R⁴ alle gleich, teilweise gleich oder unterschiedlich sind,
wobei die Reste R¹,R²,R³ und R⁴ lineare, cyclische, verzweigte, gesättigte oder ungesättigte Alkylreste, mono- oder polycyclische aromatische oder heteroaromatische Reste oder mit weiteren funktionellen Gruppen substituierte Derivate dieser Reste sind und
wobei R¹,R²,R³,R⁴ auch untereinander verbunden sein können (mehrbindiger Rest),
wobei [Yⁿ⁻] ein Hydrogencarbonat- [HCO₃⁻], Carbonat- [CO₃²⁻], Monoalkylcarbonat oder Monoarylcarbonat [ROCO₂⁻] ist, und
wobei R ein linearer, cyclischer, verzweigter, gesättigter oder ungesättigter Alkylrest, mono- oder polycyclischer Aromat oder Heteroaromat oder [ROCO₂⁻] ein anderes mono-substituiertes Carbonat ist.

Die Erfindung besteht in einem innovativen modularen Produktionssystem, mit dessen Hilfe sehr schnell und einfach aus ionischen Synthesevorstufen, den kationischen Synthesemodulen, in Kombination mit geeigneten marktüblichen Handelschemikalien, den anionische Synthesemodulen, eine extrem breite Palette an ionischen Verbindungen hergestellt werden kann, so dass eine ontime-Lieferung von industrierelevanten Mengen ermöglicht wird. Die Erfindung gestattet daher eine modulare Produktion ionischer Verbindungen bzw. deren Gemische über Vorstufen (kationische Synthesemodule) und deren Reaktion mit Handelschemikalien oder anderen lagerfähigen Zwischenprodukten (anionische Synthesemodule) in einem schnellen Syntheseschritt, der unmittelbar vor der Auslieferung erfolgen kann. Die Erfindung ermöglicht die systematische Bereitstellung einer größeren Produktpalette durch wenige Vorstufen bevorzugt in Verbindung mit ontime-lieferbaren Chemikalien. Das Herstellverfahren ist zudem ausgesprochen umweltfreundlich und in der Lage ionische Verbindungen nach Kundenwunsch und "maßgeschneidert" zur Verfügung zu stellen.

Dabei können die kationischen Synthesemodule lagerfähige Vorstufen sein, welche insbesondere zu einem späteren Zeitpunkt mit anionischen Synthesemodulen in einem modularen Produktionsprozess zu ionischen Verbindungen umgesetzt werden. Der Lageraufwand beschränkt sich daher auf universell einsetzbare Vorstufen, was angesichts kaum überschaubarer Kombinationsmöglichkeiten ein äußerst ökonomisches und flexibles System darstellt.

Gemäß einer bevorzugten Ausführungsform der Erfindung wird das kationische Synthesemodul [Q⁺]ₙ[Yⁿ⁻] in einer Br⌀nstedt-Säure/Base-Reaktion mit einer n-basigen Br⌀nstedt-Säure HₙZ als anionisches Synthesemodul versetzt, wodurch das Carbonat-, Hydrogencarbonat-, Monoalkyl- oder Monoarylcarbonat-Anion [Yⁿ⁻] als gasförmiges Kohlendioxid entweicht und gegen das gewünschte Anion [Zⁿ⁻] ersetzt wird und als Nebenprodukte Wasser, im Falle der Monoalkyl- bzw Monoarylcarbonate [ROCO₂⁻] die entsprechenden Alkohole ROH, entstehen. Dieses Verfahren ist äußerst einfach, schnell, sehr umweltfreundlich und läuft ohne jede Nebenreaktionen ab.

Bei einer weiteren, in Anspruch 8 enthaltenen Variante des Verfahrens wird in einem als "Metathese" bekannten Reaktionstyp ein geeignetes Metallsalz [M²⁺][Zⁿ⁻]_{2/n} als anionisches Synthesemodul, bevorzugt ein Strontium-, Barium-, Zink-, Mangan- oder Calciumsalz, mit der Carbonatvorstufe [Q*]₂[CO₃²⁻] als kationisches Synthesemodul umgesetzt, wobei schwerlösliche Metallcarbonat [M²⁺][CO₃²⁻] ausfällt, sodass das Carbonat-Ion der Carbonatvorstufe [Q⁺]₂[CO₃²⁻] gegen das gewünschte Anion [Zⁿ⁻] ersetzt wird. Diese Alternative kann dann von Vorteil sein, wenn bestimmte Industriechemikalien umgesetzt werden sollen.

Nach Anspruch 22 wird die gestellte Aufgabe für kationische Synthesemodule, welche durch die beispielsweise in den Ansprüchen 17 bis 21 enthaltenen Verfahren synthetisch nicht zugänglich sind, durch ein Herstellungsverfahren unter Anwendung von Flüssigionenaustausch auch gelöst.

Weitere Merkmale, Vorteile und Einzelheiten der Erfindung ergeben sich aus der nachfolgenden Beschreibung. Einige der verwendeten Begriffe sind dabei wie folgt zu verstehen:
**Ionische Flüssigkeiten:** Ionische Flüssigkeiten sind - im Sinne der anerkannten Literatur (Wasserscheid, Peter; Welton, Tom (Eds.); Ionic Liquids in Synthesis, Verlag Wiley-VCH 2003; ISBN 3-527-30515-7)- flüssige organische Salze oder Salzgemische bestehend aus organischen Kationen und organischen oder anorganischen Anionen, mit Schmelzpunkten von unter 100°C.

**Ionische Feststoffe:** Der Begriff "Ionische Feststoffe" beinhaltet in dieser Anmeldung Salze im Sinne der ionischen Flüssigkeiten, mit Schmelzpunkten von 100°C und höher. Es besteht neben diesem definitionsgemäß festgelegten Schmelzpunktbereich und dem damit verbundenen Aggregatzustand kein prinzipieller chemischer oder physikalischer Unterschied zu ionischen Flüssigkeiten.

**Ionische Verbindungen:** Sind alle ionischen Flüssigkeiten und ionischen Feststoffe im Sinne der oben angeführten Definitionen.

**Kationische Synthesemodule:** Sind die zur Produktion ionischer Verbindungen eingesetzten, bevorzugt lagerfähigen ionischen Vorstufen [Q⁺]ₙ[Yⁿ⁻], die das gewünschte Kation [Q⁺] aufweisen.

Anionische Synthesemodule: Sind die mit kationischen Synthesemodulen zur Reaktion gebrachten, marktüblichen Handelschemikalien oder anderen bevorzugt lagerfähigen Zwischenprodukte, die das Anion [Yⁿ⁻] der kationischen Synthesemodule entfernen und gegen das gewünschte Anion [Zⁿ⁻] der Ionischen Verbindung [Q⁺]ₙ[Zⁿ⁻] ersetzen.

**Synthesemodule**: Sind kationische Synthesemodule oder anionische Synthesemodule.

**Modularer Produktionsprozess**: Definiert die maßgeschneiderte Produktion ionischer Verbindungen und deren Gemische durch Kombination entsprechender kationischer und anionischer Synthesemodule (Baukastenprinzip).

Die Erfindung betrifft ein äußerst vielseitiges und beinahe universell anwendbares Produktionsverfahren zur Herstellung von ionischen Flüssigkeiten und ionischen Feststoffen. Die betreffenden ionischen Verbindungen der allgemeinen Formel [Q⁺]ₙ[Zⁿ⁻] werden in diesem neuen Verfahren in einem - im Gegensatz zu herkömmlichen Verfahren - allgemein anwendbaren, raschen und quantitativen Syntheseschritt aus einer bereits ionischen Vorstufe [Q⁺]ₙ[Yⁿ⁻] erzeugt, wobei [Yⁿ⁻] ein Hydrogencarbonat- [HCO₃⁻], Carbonat- [CO₃²⁻], Monoalkylcarbonat oder Monoarylcarbonat ([ROCO₂⁻], R = linearer, cyclischer, verzweigter, gesättigter oder ungesättigter Alkylrest, mono- oder polycyclischer Aromat oder Heteroaromat) oder irgendein anderes monosubstituiertes Carbonat darstellt. Das betreffende Kation [Q⁺]ₙ ist ein quaterniertes Ammonium- [R¹R²R³R⁴N⁺], Phosphonium- [R¹R²R³R⁴P⁺] oder Sulfonium-[R¹R²R³S⁺] Kation oder ein analoger quaternierter Stickstoff-, Phosphor- oder Schwefel-Heteroaromat (siehe Abbildung 2), wobei die Reste R¹,R²,R³,R⁴ alle gleich, teilweise gleich oder unterschiedlich sein können. Diese Reste R¹,R²,R³ und R⁴ können lineare, cyclische, verzweigte, gesättigte oder ungesättigte Alkylreste, mono- oder polycyclische aromatische oder heteroaromatische Reste oder mit weiteren funktionellen Gruppen substituierte Derivate dieser Reste sein, wobei R¹,R²,R³,R⁴ auch untereinander verbunden sein können (mehrbindiger Rest).

Die nachstehende Abbildung 2 gibt Beispiele heteroalicyclischer und heteroaromatischer Kationen [Q⁺] (unvollständige Aufzählung):

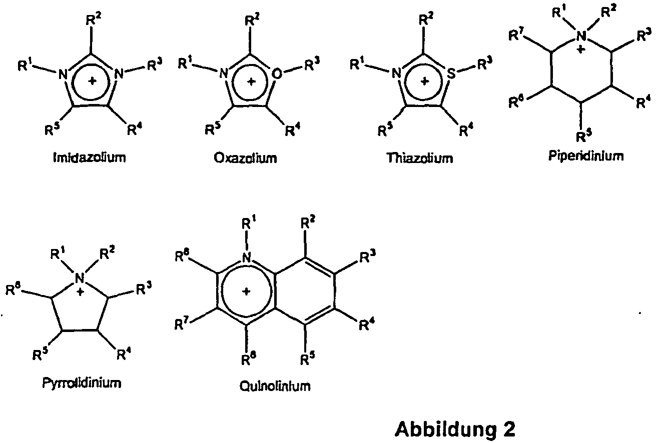

Anmerkung: Für die zusätzlichen Reste R⁵, R⁶, R⁷, R⁸ gilt dieselbe Definition wie für die beschriebene Definition der Reste R¹, R², R³, R⁴.

Die beschriebene ionische Carbonatvorstufe [Q+]ₙ[Yⁿ⁻] (kationisches Synthesemodul) ist im Allgemeinen stabil, kann in hoher Reinheit industriell produziert und gelagert werden und steht dem nun folgenden erfindungsgemäßen Produktionsprozessen jederzeit zur Verfügung. Sie kann auf zwei verschiedene und im Folgenden unter 1 a) und 1 b) genauer beschriebene Weisen zum gewünschten Produkt [Q⁺]ₙ[Zⁿ⁻] reagieren, wobei nun das Anion [Yⁿ⁻] gegen das gewünschte Anion [Zⁿ⁻] getauscht wird:

### 1a) Entfernung des Carbonat-Ions als CO₂ in einer Br⌀nstedt-Säure/Base Reaktion:

Die beschriebene ionische Vorstufe [Q⁺]ₙ[Yⁿ⁻] kann in einem bevorzugten Produktionsprozeß mit jeder beliebigen, stabilen n-basigen Br⌀nstedt-Säure (= Protonendonator) HₙZ (anionisches Synthesemodul) versetzt werden, wodurch in einer allgemein bekannten Reaktion das Carbonat- bzw. Hydrogencarbonat-Anion [Yⁿ⁻] als gasförmiges Kohlendioxid entweicht und als Nebenprodukte Wasser, im Falle der Monoalkyl- bzw Monoarylcarbonate [ROCO₂⁻] die entsprechenden Alkohole ROH, entstehen. Abbildung 3 zeigt die entsprechenden Reaktionen.

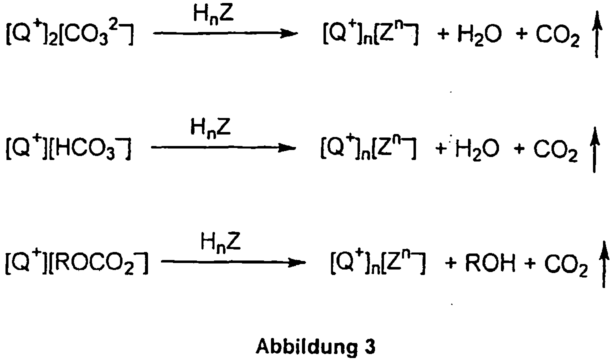

Die Reaktion kann dabei batchweise, semibatchweise oder kontinuierlich ausgeführt werden. Sie findet im Allgemeinen in wässriger oder wässrig-organischer Lösung (unter Zugabe von polaren Hilfslösungsmitteln wie niederen Alkoholen, Ketonen, Sulfoxiden, Ethern, Nitrilen) statt, kann aber, im Falle dass die ionische Vorstufe [Q⁺]ₙ[Yⁿ⁻] eine Flüssigkeit ist, auch lösungsmittelfrei ausgeführt werden. Die Zugabe der n-basigen Br⌀nstedt-Säure HₙZ kann in Substanz oder gelöst in Wasser und/oder in anderen Lösungsmitteln stöchiometrisch oder im Überschuss erfolgen, wobei insbesondere flüchtige Säuren HₙZ bevorzugt im Überschuss zugegeben werden, da sich diese später leicht im Vakuum entfernen lassen (ionische Verbindungen besitzen im Allgemeinen keinen messbaren Dampfdruck). Im Falle einer mehrbasigen Säure kann die Zugabe der n-basigen Br⌀nstedt-Säure HₙZ auch in einer solchen Stöchiometrie erfolgen, dass nicht das vollständig dissoziierte konjugierte Anion [Zⁿ⁻] entsteht, sondern auch die zugehörigen protonierten Anionen [HₘZ^{(n-m)-}] entstehen (im Falle der Phosphorsäure H₃PO₄ wären dies beispielsweise das Dihydrogenphosphat [H₂PO₄⁻] und das Hydrogenphosphat [HPO₄²⁻]). Die Säure/Basenreaktion kann durch Anlegen von Vakuum und/oder Erwärmung beschleunigt werden, wodurch eine Erhöhung der Reaktionsgeschwindigkeit und kontinuierliche Entfernung von Kohlendioxid erfolgt.

Die große Vielzahl der möglichen Br⌀nstedt-Säuren HₙZ, die aus einer einzigen, bestimmten ionischen Vorstufe [Q⁺]ₙ[Yⁿ⁻] einfach, kostengünstig, effizient und in hoher Qualität eine Vielzahl von möglichen ionischen Verbindungen [Q⁺]ₙ[Zⁿ⁻] quasi in einem "Baukastensystem" zugänglich machen, prägten den oben erwähnten Begriff "modularer Produktionsprozess", der sich von allen bis dato publizierten konventionellen Produktionsprozessen deutlich abhebt. Die dadurch zugänglichen Anionen [Zⁿ⁻] und [HₘZ^{(n-m)-}] beschränken sich nicht auf die wenigen bis jetzt üblichen Anionen wie beispielsweise Tetrafluoroborat [BF₄⁻], Hexafluorophosphat [PF₆⁻], Bis-(trifluormethylsulfonyl)imid [(CF₃SO₂)₂N⁻], Trifluormethansulfonat [CF₃SO₂⁻], Trifluoracetat [CF₃CO₃⁻], Methansulfonat [CH₃SO₂⁻], Acetat, Chlorid, Bromid, Alkylsulfat, etc. (unvollständige Aufzählung).

### 1b) Austausch des Carbonat-Ions in einer Metathesereaktion:

Eine weitere Ausführung des erfindungsgemäßen Verfahrens nützt die Schwerlöslichkeit bestimmter Metallcarbonate (wie beispielsweise Calcium-, Strontium-, Barium-, Zink- und Mangancarbonat, unvollständige Aufzählung) aus:

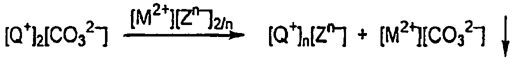

In diesem Fall wird in einem als "Metathese" bekannten Reaktionstyp (siehe "Anionenaustausch via Metathese", Beschreibungseinleitung) ein geeignetes Metallsalz [M²⁺][Zⁿ⁻]_{2/n} (bevorzugt ein Strontium-, Barium-, Zink- oder Mangansalz, besonders bevorzugt ein Calciumsalz) mit der ionischen Carbonatvorstufe [Q⁺]₂[CO₃²⁻] umgesetzt. Die treibende Kraft dieser Reaktion ist auch hier die Schwerlöslichkeit des ausfallenden Metallcarbonats [M²⁺][CO₃²⁻], allerdings sind diese Metallcarbonate (bevorzugt Strontium-, Barium-, Zink- oder Mangancarbonat, besonders bevorzugt Calciumcarbonat) bereits in wässriger Lösung schwerlöslich, sodass die gesamte Metathesereaktion in wässriger oder wässrig-organischer aber auch organischer Lösung (Verwendung von Lösungsmitteln wie Alkohole, Ketone, Ester, Sulfoxide, Ether, Nitrile, Aromaten, etc.) oder, im Falle dass die ionischen Carbonatvorstufe [Q⁺]₂[CO₃²⁻] eine Flüssigkeit ist, auch lösungsmittelfrei ausgeführt werden kann.

Dies war bei bisher üblichen Verfahren nur mit Halogenidvorstufen und sehr teuren, oft lichtempfindlichen und zum Teil toxischen Silbersalzen unter Ausfällung von schwerlöslichen Silberhalogeniden möglich oder es wurde mit kostengünstigen Metallsalzen in trockenen organischen Lösungsmitteln mit typischen Reaktionszeiten von Tagen bis Wochen wasserfrei gearbeitet (siehe "Anionenaustausch via Metathese", Beschreibungseinleitung). Beim erfindungsgemäßen Verfahren werden anstatt teurer Silbersalze kostengünstige Salze wie beispielsweise die besonders bevorzugten Calciumsalze benutzt, die Reaktion verläuft wässrig und daher schnell und einfach in der Handhabung, das beispielsweise anfallende Calciumcarbonat ist toxikologisch unbedenklich, störende Halogenidspuren werden von vornherein vermieden.

Die nachstehende Abbildung 4 zeigt die Synthese von 1-Butyl-3-methylimidazoliumlactat aus 1-Butyl-3-methylimidazoliumcarbonat und der Industriechemikalie Calciumlactat als mögliches Beispiel dieses neuen Verfahrens:

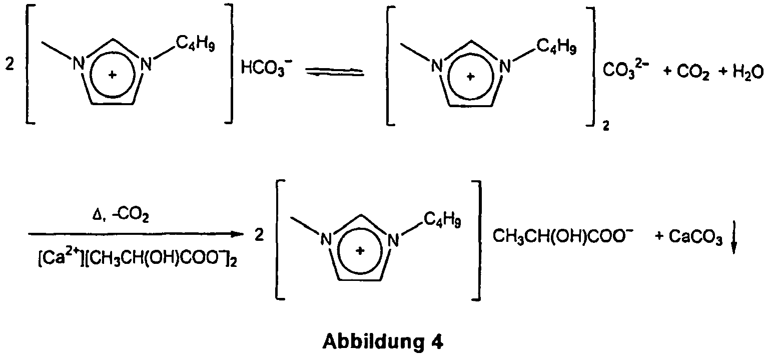

Zur Synthese der ionischen Carbonat-Vorstufe [Q⁺]ₙ[Yⁿ⁻] (kationisches Synthesemodul), welche selbst eine ionische Flüssigkeit sein kann - können die im Folgenden unter 2a) bis 2e) beschriebenen Verfahren verwendet werden, wobei die mit R,R¹,R²,R³,R⁴,R⁵, R'¹,R'²,R'³,R'⁴ beschriebenen Reste alle gleich, teilweise gleich oder unterschiedlich sein können. Diese Reste R, R¹,R²,R³,R⁴,R⁵, R'¹,R'²,R'³,R'⁴ können lineare, cyclische, verzweigte, gesättigte oder ungesättigte Alkylreste, mono- oder polycyclische aromatische oder heteroaromatische Reste oder mit weiteren funktionellen Gruppen substituierte Derivate dieser Reste sein, wobei R,R¹,R²,R³,R⁴,R⁵, R'¹,R'²R'³R'⁴ auch untereinander verbunden sein können (mehrbindiger Rest).

### 2a) Quaternierung mit organischen Kohlensäureestern:

Als Ausgangsverbindungen dieser zur Darstellung der ionischen Vorstufe [Q⁺]ₙ[Yⁿ⁻] besonders bevorzugten Synthese kommen lineare oder cyclische Amine NR¹R²R³, Phosphine PR¹R²R³, Sulfide SR¹R² oder analoge Stickstoff-, Phosphor-, Schwefel-Heteroaromaten in Frage, die in einer Substitutionsreaktion einen organischen Kohlensäureester R⁴O(CO)OR⁵ nucleophil angreifen (siehe nachstehende Abbildung 5):

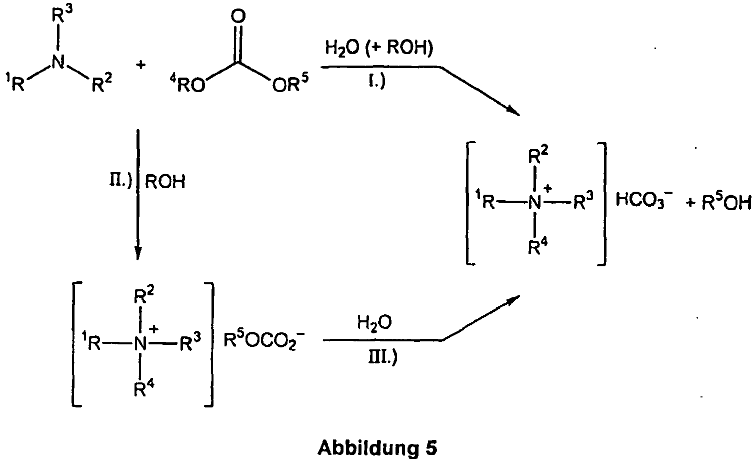

Am Beispiel eines Amins NR¹R²R³ bildet sich in wässriger Lösung (siehe I. in Abbildung 5) direkt ein entsprechendes Ammoniumhydrogencarbonat [R¹R²R³R⁴N⁺][HCO₃⁻]. Als Lösungsvermittler kann hier bei Bedarf zusätzlich zum Wasser ein polares Lösungsmittel, wie niedermolekulare Alkohole, Dimethylformamid, Acetonitrol etc., zugegeben werden; eine zweiphasige, rein wässrige Reaktionsführung unter starkem Rühren ist aber ebenfalls möglich.

Führt man die Reaktion in ebengenannten polaren Lösungsmitteln ohne Zusatz von Wasser aus (siehe II. in Abbildung 5), erhält man das entsprechende Ammoniummonoalkyl- bzw. Ammoniummonoarylcarbonat [R¹R²R³R⁴N⁺[R⁵OCO₂⁻]. Es lässt sich entweder isolieren oder durch Hydrolyse (siehe III.) in das entsprechende Ammoniumhydrogencarbonat [R¹R²R³R⁴N⁺[HCO₃⁻] überführen. Die industrielle Synthese von hochreinen, quaternären Ammoniumhydrogencarbonaten zur elektrochemischen Gewinnung von quaternären Ammoniumhydroxiden ist beispielsweise im US Patent Nr. 4776929 beschrieben.

Im Gegensatz zu den bis dato üblichen Quaternierungsreagentien wie Alkyl- oder Arylhalogenide, Dialkyl- oder Diarylsulfate etc. (siehe "Alkylierung bzw. Arylierung zu einem quaternären Salz", in der Beschreibungseinleitung) sind die verwendeten organischen Carbonate R⁴O(CO)OR⁵wie beispielsweise Diethylcarbonat, Dibutylcarbonat oder das cyclische 1,3-Dioxolan-2-on (unvollständige Aufzählung) human- und umwelttoxikologisch unbedenklich (WGK1, CH-Giftklasse: Frei, nicht atmosphärenschädigend) und leicht entsorgbar.

### 2b) Allgemeine Metathese mit Carbonaten und Hydrogencarbonaten:

Manche ionische Vorstufen [Q⁺]ₙ[Yⁿ⁻], welche durch Quaternierung mit organischen Kohlensäureestern nicht zugänglich sind, können durch die bereits beschriebene Metathesereaktion (siehe d. "Anionenaustausch via Metathese", Beschreibungseinleitung) hergestellt werden, wie Abbildung 6 zeigt:

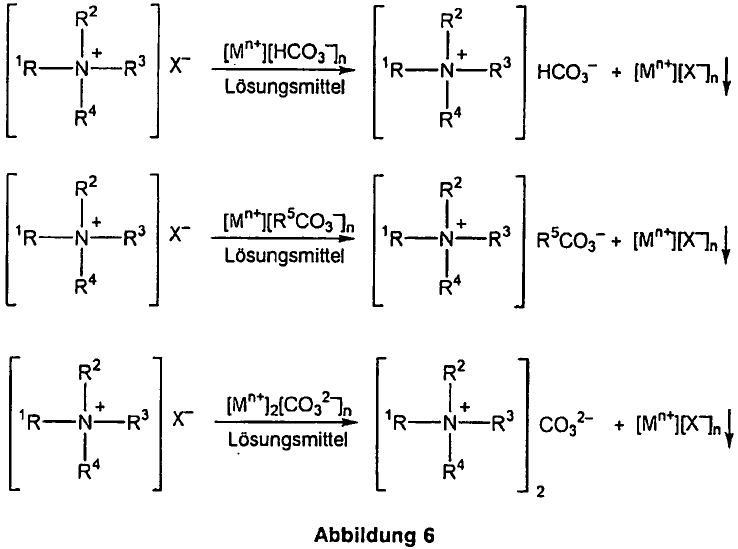

Am Beispiel einer quaternären Ammoniumverbindung [R¹R²R³R⁴N⁺][X⁻], welche durch Alkylierung oder Arylierung eines tertiären Amins R¹R²R³N erhalten wurde (siehe"Alkylierung bzw. Arylierung zu einem quaternären Salz", in der Beschreibungseinleitung), kann nun in einem geeigneten, trockenen Lösungsmittel unter Ausschluss von Luftfeuchtigkeit ein Hydrogencarbonat [Mⁿ⁺][HCO₃⁻]ₙ, ein Carbonat [Mⁿ⁺]₂[CO₃²⁻]ₙ oder ein Monoalkyl- bzw. Monoarylcarbonat [Mⁿ⁺][R⁵CO₃⁻]ₙ zugegeben werden, wobei [Mⁿ⁺] ein geeignetes Metallkation darstellt. Unter Rühren bildet sich die gewünschte ionische Verbindung in Form eines quaternären Ammoniumhydrogencarbonats [R¹R²R³R⁴N⁺][HCO₃⁻], eines quaternären Ammoniumcarbonats [R¹R²R³R⁴N⁺]₂[CO₃²⁻] oder eines quaternären Ammoniummonoalkyl- bzw. Ammoniummonoarylcarbonats [R¹R²R³R⁴N⁺][R⁵CO₃⁻], wobei das entsprechende Alkali- oder Erdalkalisalz [Mⁿ⁺][X⁻] als Feststoff ausfällt und durch Filtration entfernt wird. Die gewünschte ionische Verbindung wird dann durch Abdestillieren des Lösungsmittels gewonnen.

### 2c) Spezielle Sulfat-Metathese mit Erdalkalicarbonaten:

Erdalkalisulfate (mit Ausnahme des Magnesiumsulfats) sind in Wasser schwer löslich (Löslichkeiten in g/100g Wasser. CaSO₄ 0,205 (25°C); SrSO₄ 0,0135 (25°C); BaSO₄0,00031 (20°C) Quelle: CRC Handbook of Chemistry and Physics, 83rd Edition, 2002, ISBN 0-8493-0483-0), während Erdalkalihydrogencarbonate verhältnismäßig gute Löslichkeit zeigen (CD Römpp Chemie Lexikon - Version 1.0, Stuttgart/New York: Georg Thieme Verlag 1995). Dieses Ionensystem eignet sich daher hervorragend, um in einer speziellen Metathesereaktion quaternäre Ammonium-, Phosphonium-, Sulfonium oder analoge heteroaromatische Hydrogencarbonate [Q]⁺[HCO₃⁻] herzustellen. Die entsprechenden Erdalkalihydrogencarbonate können in wässriger Lösung leicht aus den schwerlöslichen Erdalkalicarbonaten durch Reaktion mit Kohlendioxid erzeugt werden, sind aber als isolierte Feststoffe unbekannt. Als Ausgangssubstanzen dieser Metathese dienen in diesem Fall quaternäre Ammonium-, Phosphonium-, Sulfonium oder analoge heteroaromatische Sulfate [Q]₂⁺[SO₄²⁻], welche durch Alkylierung- oder Arylierung von linearen oder cyclischen Aminen NR¹R²R³, Phosphinen PR¹R²R³, Sulfiden SR¹R² oder analogen Stickstoff-, Phosphor-, Schwefel-Heteroaromaten mittels organischen Sulfaten R⁴O(SO₂)OR⁵ und nachfolgender Hydrolyse leicht zugänglich sind:

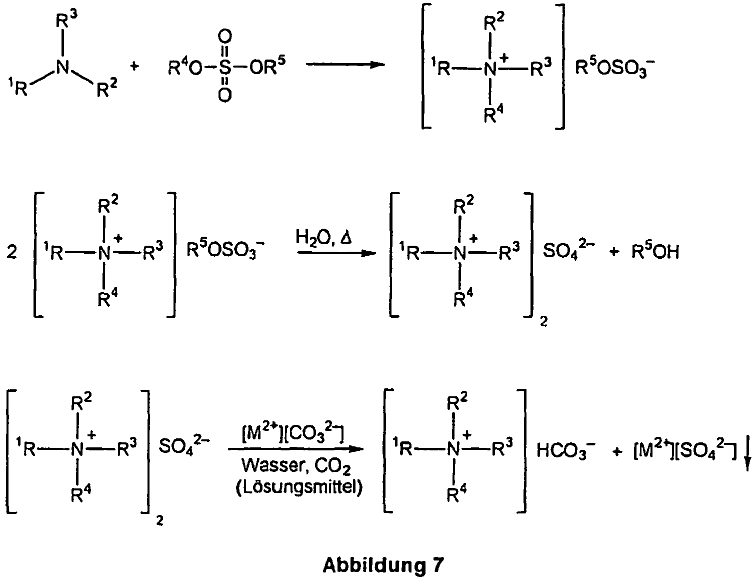

Abbildung 7 zeigt am Beispiel eines Amins NR¹R²R³ eine typische Synthese: Das tertiäre Amin wird in einer allgemein bekannten Reaktion mit einem Alkyl- oder Arylsulfat R⁴O(SO₂)OR⁵ umgesetzt. Dieses starke Alkylierungs- bzw. Arylierungsmittel quaterniert das Amin zum entsprechenden Ammoniummonoalkylsulfat bzw Ammoniummonoarylsulfat [R¹R²R³R⁴N⁺][R⁵SO₄⁻], welches in einem nachfolgenden Schritt in wässriger Lösung (wenn erforderlich unter Zugabe von polaren Lösungsmitteln) gänzlich in das entsprechende Sulfat [R¹R²R³R⁴N⁺]₂[SO₄²⁻] hydrolisiert wird. Der dabei entstehende Alkohol R⁵OH kann im Reaktionsgemisch verbleiben oder destillativ entfernt werden. Bei Temperaturen > 100°C reagieren niedermolekulare Sulfate R⁴O(SO₂)OR⁴ wie z.B. Dimethylsulfat auch direkt zum entsprechenden Ammoniumsulfat [R¹R²R³R⁴N⁺]₂[SO₄²⁻], sodass der Hydrolyseschritt entfällt.

Das so dargestellte Ammoniumsulfat [R¹R²R³R⁴N⁺]₂[SO₄²⁻] kann im Reaktionsgefäß verbleiben und wird nun in wässriger Lösung (bzw. wässrig-organischer Lösung, falls ein polares Hilfslösungsmittel benötigt wird, um es in Lösung zu bringen) mit einem Erdalkalicarbonat [M²⁺][CO₃²⁻] versetzt, wobei bevorzugt Calciumcarbonat, aber auch Strontium- und Bariumcarbonat verwendet werden können. Unter kräftigem Rühren wird gasförmiges Kohlendioxid oder festes Kohlendioxid ("Trockeneis") zugeführt, wobei die schwerlöslichen Erdalkalicarbonate in die entsprechenden leichtlöslichen Erdalkalihydrogencarbonate [M²⁺][HCO₃⁻]₂ überführt werden.

Die dadurch freigesetzten, gelösten Calcium-, Strontium- oder Bariumkationen reagieren dabei sofort und stetig (entsprechend der Geschwindigkeit der Hydrogencarbonatbildung) mit den vorhanden Sulfationen und bilden schwertlösliches Calcium-, Strontium- oder Bariumsulfat, das als Feststoff ausfällt. Nach Beendigung der Reaktion und Entfernung des ausgefallenen Erdalkalisulfates [M²⁺][SO₄²⁻] durch Filtration, kann das entsprechende Ammoniumhydrogencarbonat [R¹R²R³R⁴N⁺][HCO₃⁻] durch Abdestillieren des Wasser-Lösungsmittelgemisches isoliert werden, wobei es nach dem bekannten Gleichgewicht 2 [HCO₃⁻] →[CO₃²⁻] + CO₂ + H₂O durch Entweichen des Kohlendioxids teilweise oder ganz in das entsprechende Ammoniumcarbonat [R¹R²R³R⁴N⁺]₂[CO₃²⁻] überführt wird.

### 2d) Reaktion von Hydroxiden und Alkoholaten mit Kohlendioxid:

Eine weitere Möglichkeit zur Synthese der ionischen Vorstufe [Q⁺]ₙ[Yⁿ⁻] stellt die Reaktion von quaternären Hydroxiden [Q⁺][OH⁻] und Alkoholaten [Q⁺][RO⁻] mit Kohlendioxid dar (Synthesis, (1), 33-4; 1985 und Journal of Organic Chemistry, 67(23), 8287-8289; 2002). Im Falle der Hydroxide kommt dabei als Lösungsmittel Wasser und / oder ein polares Lösungsmittel zur Anwendung, im Falle der Alkoholate nur ein polares und aprotisches Lösungsmittel, wenn die entsprechenden Monoalkyl- oder Monoarylcarbonate [Q⁺][ROCO₂⁻] dargestellt werden sollen, denn die Alkoholate reagieren ansonsten zu Hydroxiden und den freien Alkoholen nach [Q⁺][RO⁻] + H₂O → [Q⁺][OH⁻] + ROH, die allerdings wieder mit CO₂ zu den Hydrogencarbonaten [Q⁺][HCO₃⁻] umgesetzt werden können.

Am Beispiel eines quaternären Ammoniumhydroxids [R¹R²R³R⁴N⁺][OH⁻] bzw. Ammoniumalkoholates [R¹R²R³R⁴N⁺][R⁵O⁻] zeigt Abbildung die Synthese des quaternären Ammoniumhydogencarbonats [R¹R²R³R⁴N⁺][HCO₃⁻] bzw. des Ammoniummonoalkylcarbonats bzw. Ammoniummonoarylcarbonats [R¹R²R³R⁴N⁺][R⁵OCO₂⁻].

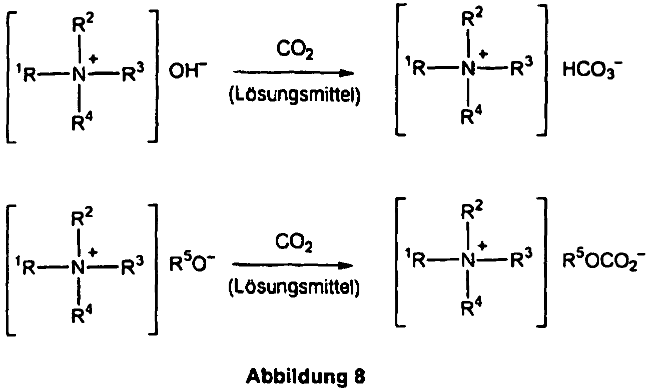

### 2e) Flüssigionenaustausch mit mittel- bis langkettigen quaternären Carbonaten

Mittel- bis langkettige quaternäre Verbindungen [Q'⁺]ₙ[Yⁿ⁻], die z.B. in einem der zuvor beschriebenen Prozesse 2a - 2d hergestellt worden sind, wobei
[Q'⁺l ein quaterniertes Ammonium- [R'¹R'²R'³R'⁴N⁺], Phosphonium- [R'¹R'²R'³R'⁴P⁺] oder Sulfonium- [R'¹R'²R'³S⁺] Kation oder ein analoger quaternierter Stickstoff-, Phosphor- oder Schwefel-Heteroaromat ist,
wobei die Reste R'¹,R'²,R'³,R'⁴ alle gleich, teilweise gleich oder unterschiedlich sind,
wobei die Reste R'¹,R'²,R'³ und R'⁴ lineare, cyclische, verzweigte, gesättigte oder ungesättigte Alkylreste, mono- oder polycyclische aromatische oder heteroaromatische Reste oder mit weiteren funktionellen Gruppen substituierte Derivate dieser Reste sind und
wobei R'¹,R'²,R'³,R'⁴ auch untereinander verbunden sein können (mehrbindiger Rest),
wobei mindestens einer der Reste R'¹,R'²,R'³,R'⁴ 4 - 30 Kohlenstoffatome aufweist,
wobei [Yⁿ⁻]ein Hydrogencarbonat- [HCO₃⁻], Carbonat- [CO₃²⁻], Monoalkylcarbonat oder Monoarylcarbonat [R'OCO₂⁻] ist, und
wobei R' ein linearer, cyclischer, verzweigter, gesättigter oder ungesättigter Alkylrest, mono- oder polycyclischer Aromat oder Heteroaromat oder [R'OCO₂⁻] ein anderes mono- substituiertes Carbonat ist,
zeigen in wäßriger oder in wäßrig-organischer Lösung (Zusatz von polaren Hilfslösungsmitteln wie niederen Alkoholen, Ketonen, Sulfoxiden, Ethern, Nitrilen etc.) die Fähigkeit zum Flüssigionenaustausch: Diese mittel- bis langkettigen quaternären Verbindungen sind aufgrund der stark polaren, hydrophilen Carbonat-Anionen [Yⁿ⁻] noch immer gut bis mäßig wasserlöslich, während entsprechende Verbindungen [Q'⁺]ₙ[Xⁿ⁻] mit geeigneter Kettenlänge im Allgemeinen unpolare, hydrophobe und daher wasserunmischbare Verbindungen sind, wobei
[Xⁿ⁻] z.B. ein Chlorid-, Bromid-, Iodid-, Sulfat-, Phosphat-, p-Toluolsulfonat-, Methansulfonat-, Trifluormethansulfonat-, Trifluoracetat-Anion (unvollständige Aufzählung) oder jedes andere Anion sein kann, welches mit - wie bereits beschrieben - mit Kationen [Q⁺] Wasser unmischbare Verbindungen bildet.

Dieser Umstand kann zur Synthese ionischer Vorstufen [Q⁺]ₙ[Yⁿ⁻] (siehe weiter vorne in der Beschreibung) ausgenutzt werden, welche z.B. mit den Methoden 2a - 2d synthetisch nicht zugänglich sind:
Dabei wird eine entsprechende mittel- bis langkettige quaternäre Verbindung [Q'⁺]ₙ[Yⁿ⁻] mit einer quaternären Verbindung [Q⁺]ₙ[Xⁿ⁻] in wäßriger oder wäßrig-organischer Lösung umgesetzt, die durch klassische, allgemein bekannte Quaternierung eines entsprechenden linearen oder cyclischen Amins NR¹R²R³, Phosphins PR¹R²R³, Sulfids SR¹R² oder analogen Stickstoff-, Phosphor-, Schwefel-Heteroaromaten mit z.B. einem Alkyl- oder Aryl-Halogenid, p-Toluolsulfonat, Methansulfonat, Trifluormethansulfonat, Trifluoracetat, oder Dialkylsulfat oder Diarylsulfat (unvollständige Aufzählung) hergestellt wurde und die das gewünschte Kation [Q⁺] trägt:

[Q'⁺]ₙ[Yⁿ⁻] + [Q⁺]ₙ[Xⁿ⁻] → [Q⁺]ₙ[Yⁿ⁻] + [Q'⁺]ₙ[Xⁿ⁻]

Nach der Reaktion scheidet sich die Verbindung [Q'⁺]ₙ[Xⁿ⁻] als flüssige, wasserunmischbare Phase (oder- je nach Temperatur- als kristalliner oder wachsartiger Feststoff) ab und die gewünschte ionische Vorstufe [Q⁺]ₙ[Yⁿ⁻] bleibt in der wäßrigen Phase zurück, aus der sie (nach Abtrennung der Verbindung [Q'⁺]ₙ[Xⁿ⁻]) durch Entfernung des Wassers bzw. Wasser-Lösungsmittelgemisches (Verdampfen, Destillation) isoliert werden kann.

Ein wesentlicher Vorteil dieses Verfahrens liegt - ähnlich dem Verfahren 2c. "Spezielle Sulfat-Metathese mit Erdalkalicarbonaten" - in der Anwendung von Wasser bzw. wässrigorganischen Lösungsmittelsystemen: Es muss nicht (wie z.B. im Falle des in der Beschreibung des Standes der Technik unter d. aufgeführtem "Anionenaustausch durch Metathese") unter Ausschluss von Luftfeuchtigkeit gearbeitet werden und darüber hinaus ist die Brandsicherheit wesentlich erhöht, sodass insbesondere eine industrielle Produktion deutlich erleichtert wird.

## Patentansprüche

1. Verfahren zur Herstellung ionischer Flüssigkeiten, ionischer Feststoffe oder Gemische derselben aus kationischen Synthesemodulen [Q⁺]ₙ[Yⁿ⁻] und anionischen Synthesemodulen,
wobei [Q⁺] ein quaterniertes Ammonium- [R¹R²R³R⁴N⁺], Phosphonium- [R¹R²R³R⁴P⁺] oder Sulfonium- [R¹R²R³S⁺] Kation oder ein analoger quaternierter Stickstoff-, Phosphor- oder Schwefel-Heteroaromat ist,
wobei die Reste R¹,R²,R³,R⁴ alle gleich, teilweise gleich oder unterschiedlich sind,
wobei die Reste R¹,R²,R³ und R⁴ lineare, cyclische, verzweigte, gesättigte oder ungesättigte Alkylreste, mono- oder polycyclische aromatische oder heteroaromatische Reste oder mit weiteren funktionellen Gruppen substituierte Derivate dieser Reste sind und
wobei R¹,R²,R³,R⁴ auch untereinander verbunden sein können (mehrbindiger Rest), wobei [Yⁿ⁻] ein Hydrogencarbonat- [HCO₃], Carbonat- [CO₃²⁻], Monoalkylcarbonat oder Monoarylcarbonat ([ROCO₂⁻] ist und
wobei R ein linearer, cyclischer, verzweigter, gesättigter oder ungesättigter Alkylrest, mono- oder polycyclischer Aromat oder Heteroaromat oder [ROCO₂⁻] ein anderes mono- substituiertes Carbonat ist.

2. Verfahren nach Anspruch 1, **dadurch gekennzeichnet, dass** bevorzugt lagerfähige kationische Synthesemodule mit anionischen Synthesemodulen in einem modularen Produktionsprozess zu ionischen Verbindungen umgesetzt werden.

3. Verfahren nach Anspruch 1 oder 2, **dadurch gekennzeichnet, dass** das kationische Synthesemodul [Q⁺]ₙ[Yⁿ⁻] in einer Brønstedt-Säure/Base-Reaktion mit einer n-basigen Brønstedt-Säure HₙZ als anionisches Synthesemodul versetzt wird, wodurch das Carbonat-, Hydrogencarbonat-, Monoalkyl- oder Monoarylcarbonat-Anion [Yⁿ⁻] als gasförmiges Kohlendioxid entweicht und gegen das gewünschte Anion [Zⁿ⁻] ersetzt wird und als Nebenprodukte Wasser, im Falle der Monoalkyl- bzw Monoarylcarbonate [ROCO₂⁻] die entsprechenden Alkohole ROH, entstehen.

4. Verfahren nach einem der Ansprüche 1 bis 3, **dadurch gekennzeichnet, dass** die Zugabe der n-basigen Brønstedt-Säure HₙZ in Substanz oder gelöst in Wasser und/oder anderen Lösungsmitteln stöchiometrisch oder im Überschuss erfolgt.

5. Verfahren nach einem der Ansprüche 1 bis 4, **dadurch gekennzeichnet, dass** die Zugabe der n-basigen Brønstedt-Säure HₙZ auch in einer solchen Stöchiometrie erfolgen kann, dass nicht das vollständig dissoziierte konjugierte Anion [Zⁿ⁻] entsteht, sondern die zugehörigen protonierten Anionen [HₘZ^{(n-m)-}], m < n.

6. Verfahren nach einem der Ansprüchen 1 bis 5, **dadurch gekennzeichnet, dass** die Reaktion in wässrigem, wässrig/organischem, organischem Lösungsmittel (Zugabe von polaren Lösungsmitteln wie niederen Alkoholen, Ketonen, Sulfoxiden, Ethern, Nitriten) oder - im Falle dass das kationische Synthesemodul [Q⁺]ₙ[Yⁿ⁻] flüssig ist - auch lösungsmittelfrei durchgeführt wird.

7. Verfahren einem der Ansprüchen 1 bis 6, **dadurch gekennzeichnet, dass** die Säure/Basenreaktion durch Anlegen von Vakuum beschleunigt wird.

8. Verfahren nach Anspruch 1 oder 2, **dadurch gekennzeichnet, dass** in einem als "Metathese" bekannten Reaktionstyp ein geeignetes Metallsalz [M²⁺][Zⁿ⁻]_{2/n} als anionisches Synthesemodul, bevorzugt ein Strontium-, Barium-, Zink-, Mangan- oder Calciumsalz, mit der Carbonatvorstufe [Q⁺]₂[CO₃²⁻] als kationisches Synthesemodul umgesetzt wird, wobei schwerlösliches Metallcarbonat [M²⁺][CO₃²⁻] ausfällt, sodass das Carbonat-Ion der Carbonatvorstufe [Q⁺]₂[CO₃²⁻] gegen das gewünschte Anion [Zⁿ] ersetzt wird.

9. Verfahren nach Anspruch 1, 2 oder 8, **dadurch gekennzeichnet, dass** das ausgefallene schwerlösliche Metallcarbonat [M²⁺][CO₃²⁻] durch Filtration, Zentrifugation oder andere geeignete Verfahren von der gewünschten ionischen Verbindung [Q⁺]ₙ[Zⁿ⁻] getrennt wird.

10. Verfahren nach einem der Ansprüche 1, 2, 8 oder 9, **dadurch gekennzeichnet, dass** die Reaktion in wässrigem, wässrig/organischem oder organischem Lösungsmittel (Zugabe von polaren Lösungsmitteln wie niederen Alkoholen, Ketonen, Sulfoxiden, Ethern, Nitrilen) oder - im Falle dass das kationische Synthesemodul [Q⁺]₂[CO₃²⁻] und die gewünschte ionische Verbindung [Q⁺]ₙ[Zⁿ⁻] flüssig sind - auch lösungsmittelfrei durchgeführt wird.

11. Verfahren nach einem der Ansprüche 3 bis 7, **dadurch gekennzeichnet, dass** die Reaktion batchweise, semibatchweise oder kontinuierlich durchgeführt wird.

12. Verfahren nach einem der Ansprüche 1 bis 10, **dadurch gekennzeichnet, dass** das Reaktionsgemisch gerührt, geschüttelt oder anderweitig durchmischt wird.

13. Verfahren nach einem der Ansprüche 1 bis 12, **dadurch gekennzeichnet, dass** nach beendeter Reaktion das Lösungsmittel durch Destillation, Vakuumdestillation, Dünnschichtverdampfung, Rotationsverdampfung oder andere geeignete Verfahren entfernt wird.

14. Verfahren nach einem der Ansprüche 1 bis 13, **dadurch gekennzeichnet, dass** handelsübliche Industriechemikalien verwendet werden.

15. Verfahren nach einem der Ansprüche 1 bis 14, **dadurch gekennzeichnet, dass** als Lösungsmittel wässrige Systeme verwendet werden.

16. Verfahren nach einem der Ansprüche 1 bis 15, **dadurch gekennzeichnet. dass** die lagerfähigen kationische Synthesemodule erst unmittelbar vor der Produktion der ionischen Verbindung mit den anionischen Synthesemodulen umgesetzt werden.

17. Verfahren nach nach einem der Ansprüche 1 bis 16, **dadurch gekennzeichnet, dass** keine Halogen- und / oder Silberverbindungen zur Anwendung kommen.

18. Verfahren nach einem der Ansprüche 1 bis 17, **dadurch gekennzeichnet, dass** die Ionische Carbonatvorstufe [Q⁺]ₙ[Yⁿ⁻] (kationisches Synthesemodul) durch Quaternierung von linearen oder cyclischen Aminen NR¹R²R³, Phosphinen PR¹R²R³, Sulfiden SR¹R² oder analogen Stickstoff-, Phosphor-, Schwefel-Heteroaromaten in einer nucleophilen Substitutionsreaktion mit organischen Kohlensäureestern R⁴O(CO)OR⁵ erzeugt wird, wobei die Reste R¹,R²,R³,R⁴,R⁵ alle gleich, teilweise gleich oder unterschiedlich sind und wobei diese Reste lineare, cyclische, verzweigte, gesättigte oder ungesättigte Alkylreste, mono- oder polycyclische aromatische oder heteroaromatische Reste oder mit weiteren funktionellen Gruppen substituierte Derivate dieser Reste sind, wobei jeweils R¹,R²,R³ und R⁴,R⁵ auch untereinander verbunden sein können (mehrbindiger Rest).

19. Verfahren einem der Ansprüche 1 bis 18, **dadurch gekennzeichnet, dass** die ionische Carbonatvorstufe [Q⁺]ₙ[Yⁿ⁻] (kationisches Synthesemodul) durch "Metathese" aus einer bereits ionischen Vorstufe [Q⁺]ₙ[Xⁿ⁻] erzeugt wird, wobei in einem geeigneten, trockenen Lösungsmittel unter Ausschluß von Luftfeuchtigkeit ein Hydrogencarbonat [Mⁿ⁺][HCO₃⁻]ₙ, ein Carbonat [Mⁿ⁺]₂[CO₃²⁻]ₙ oder ein Monoalkyl- bzw. Monoarylcarbonat [Mⁿ⁺][RCO₃⁻]ₙ zugegeben wird, wobei [Mⁿ⁺] ein geeignetes Metallkation und R ein linearer, cyclischer, verzweigter, gesättigter oder ungesättigter Alkylrest, ein mono- oder polycyclischer aromatischer oder heteroaromatischer Rest oder ein mit weiteren funktionellen Gruppen substituiertes Derivat dieser Reste ist, wobei unter Rühren die gewünschte ionische Verbindung [Q⁺]ₙ[Yⁿ⁻] (kationisches Synthesemodul) gebildet wird, wobei das entsprechende Alkali- oder Erdalkalisalz [Mⁿ⁺][X⁻] als Feststoff ausfällt und insbesondere durch Filtration entfernt wird.

20. Verfahren nach einem der Ansprüche 1 bis 17 oder 19, **dadurch gekennzeichnet, dass** [Yⁿ⁻] ein Hydrogencarbonat [HCO₃⁻] ist, dass [Xⁿ⁻] ein Sulfat [SO₄²⁻] ist und dass [Mⁿ⁺] ein Strontium oder Barium, besonders bevorzugt ein Calciumkation ist, wobei das quaternäre Ammonium-, Phosphonium-, Sulfonium oder analoge heteroaromatische Sulfat [Q]₂⁺[SO₄²⁻] durch Alkylierung- oder Arylierung von linearen oder cyclischen Aminen NR¹R²R³, Phosphinen PR¹R²R³, Sulfiden SR¹R² oder analogen Stickstoff-, Phosphor-, Schwefel-Heteroaromaten mittels organischen Sulfaten R⁴O(SO₂)OR⁵ erzeugt wurde und wobei das zum Sulfat [Q]₂⁺[SO₄²⁻] zugesetzte Metallsalz ein Hydrogencarbonat [Mⁿ⁺][HCO₃⁻]₂ ist, welches zuvor oder in situ in wässriger oder wässrig-organischer Lösung durch Reaktion des entsprechenden schwerlöslichen Erdalkalicarbonats [Mⁿ⁺][CO₃²⁻] mittels Kohlendioxid erzeugt wurde, wobei das ausfallende, schwerlösliche Strontium-, Barium- oder Calciumsulfat insbesondere durch Filtration entfernt wird und auch die Reste R¹,R²,R³,R⁴,R⁵ alle gleich, teilweise gleich oder unterschiedlich sind und wobei diese Reste lineare, cyclische, verzweigte, gesättigte oder ungesättigte Alkylreste, mono- oder polycyclische aromatische oder heteroaromatische Reste oder mit weiteren funktionellen Gruppen substituierte Derivate dieser Reste sind, wobei jeweils R¹,R²,R³ und R⁴,R⁵ auch untereinander verbunden sein können (mehrbindiger Rest).

21. Verfahren nach einem der Ansprüche 1 bis 17, **dadurch gekennzeichnet, dass** die ionische Carbonatvorstufe [Q⁺]ₙ[Yⁿ⁻] (kationisches Synthesemodul) durch Reaktion von quaternären Hydroxiden [Q⁺][OH⁻] und Alkoholaten [Q⁺][RO⁻] mit Kohlendioxid hergestellt wird, wobei R ein linearer, cyclischer, verzweigter, gesättigter oder ungesättigter Alkylrest, ein mono- oder polycyclischer aromatischer oder heteroaromatischer Rest oder ein mit weiteren funktionellen Gruppen substituiertes Derivat dieses Restes ist.

22. Verfahren nach einem der Ansprüche 1 bis 17, **dadurch gekennzeichnet, dass** die ionische Carbonatvorstufe [Q⁺]ₙ[Yⁿ⁻] (kationisches Synthesemodul) durch Flüssigionenaustausch zwischen einer mittel- bis langkettigen quaternären Verbindung [Q'⁺]ₙ[Yⁿ⁻] und einer quaternären Verbindung [Q⁺]ₙ[Xⁿ⁻] in wäßriger oder wäßrig-organischer Lösung nach
[Q'⁺]ₙ[Yⁿ⁻] + [Q⁺]ₙ[Xⁿ⁻] → [Q⁺]ₙ[Yⁿ⁻] + [Q'⁺]ₙ[Xⁿ⁻] erfolgt, wobei
[Q'⁺] ein quaterniertes Ammonium- [R'¹R'²R'³R'⁴N⁺], Phosphonium- [R'¹R'²R'³R'⁴P⁺] oder Sulfonium- [R'¹R'²R'³S⁺] Kation oder ein analoger quaternierter Stickstoff-, Phosphor- oder Schwefel-Heteroaromat ist,
wobei die Reste R'¹R'²R'³R'⁴ alle gleich, teilweise gleich oder unterschiedlich sind,
wobei die Reste R'¹R'²R'³ und R'⁴ lineare, cyclische, verzweigte, gesättigte oder ungesättigte Alkylreste, mono- oder polycyclische aromatische oder heteroaromatische Reste oder mit weiteren funktionellen Gruppen substituierte Derivate dieser Reste sind und
wobei R'¹R'²R'³R'⁴ auch untereinander verbunden sein können (mehrbindiger Rest),
wobei mindestens einer der Reste R'¹R'²R'³R'⁴ 4-30 Kohlenstoffatome aufweist,
wobei [Yⁿ⁻] ein Hydrogencarbonat- [HCO₃⁻], Carbonat- [CO₃²⁻], Monoalkylcarbonat oder Monoarylcarbonat [R'OCO₂⁻] ist, und
wobei R' ein linearer, cyclischer, verzweigter, gesättigter oder ungesättigter Alkylrest, mono- oder polycyclischer Aromat oder Heteroaromat oder [R'OCO₂⁻] ein anderes mono-substituiertes Carbonat ist,
wobei [Q⁺] das gewünschte Kation der ionischen Vorstufe ist,
wobei [Xⁿ⁻] ein Chlorid-, Bromid-, Iodid-, Sulfat-, Phosphat-, p-Toluolsulfonat-, Methansulfonat-, Trifluormethansulfonat-, Trifluoracetat-Anion oder jedes andere Anion sein kann, welches aus einer klassischen Quaternierungsreaktion eines entsprechenden linearen oder cyclischen Amins NR¹R²R³, Phosphins PR¹R²R³, Sulfids SR¹R² oder analogen Stickstoff-, Phosphor-, Schwefel-Heteroaromaten hervorgeht,
wobei [Q'⁺]ₙ[Xⁿ⁻] sich als wasserunmischbare Phase nach erfolgtem Flüssigionenaustausch abtrennt,
wobei die gewünschte ionische Vorstufe [Q⁺]ₙ[Yⁿ⁻] nach Separation der wasserunmischbaren Phase [Q'⁺]ₙ[Xⁿ⁻] in wäßriger oder wäßrig-organischer Lösung vorliegt,
und wobei sich diese Vorstufe [Q'⁺]ₙ[Yⁿ⁻] schließlich durch Entfernung des Lösungsmittels (Verdampfen, Destillation) gewinnen läßt.

## Claims

1. A method for producing ionic liquids, ionic solids or mixtures of same from cationic synthesis modules [Q⁺]ₙ[Yⁿ⁻] and anionic synthesis modules,
wherein [Q⁺] is a quaternized ammonium cation [R¹R²R³R⁴N⁺], phosphonium cation [R¹R²R³R⁴P⁺] or sulfonium cation [R¹R²R³S⁺] or a similar quaternized nitrogen, phosphorus or sulfur heteroaromatic,
wherein the radicals R¹, R², R³, R⁴ are all the same, partially the same or different,
wherein the radicals R¹, R², R³ and R⁴ are linear, cyclic, branched, saturated or unsaturated alkyl radicals, monocyclic or polycyclic aromatic or heteroaromatic radicals or derivatives of these radicals substituted with additional functional groups, and
wherein R¹, R², R³, R⁴ may also be interconnected (multibond radical),
wherein [Yⁿ⁻] is a bicarbonate [HCO³⁻], carbonate [CO₃²⁻], monoalkyl carbonate or monoaryl carbonate [ROCO₂⁻], and
wherein R is a linear cyclic branch saturated or unsaturated alkyl radical, monocyclic or polycyclic aromatic or heteroaromatic or [ROCO₂⁻] is another monosubstituted carbonate.

2. The method according to Claim 1,
**characterized in that**
preferably storable cationic synthesis modules are reacted with anionic synthesis modules in a modular production process to form ionic compounds.

3. The method according to Claim 1 or 2,
**characterized in that**
the cationic synthesis module [Q⁺]ₙ[Yⁿ⁻] is mixed with an n-base Brønstedt acid HₙZ as the anionic synthesis module in a Brønstedt acid-base reaction, so that the carbonate, bicarbonate, monoalkyl or monoaryl carbonate anion [Yⁿ⁻] escapes as gaseous carbon dioxide and is replaced by the desired anion [Zⁿ⁻], and the byproducts formed include water, and in the case of the monoalkyl and/or monoaryl carbonates [ROCO₂⁻], the corresponding alcohols ROH are also formed.

4. The method according to any one of Claims 1 to 3,
**characterized in that**
the n-base Brønstedt acid HₙZ is added in substance or dissolved in water and/or in other solvents in a stoichiometric ratio or in excess.

5. The method according to any one of Claims 1 to 4,
**characterized in that**
the n-base Brønstedt acid HₙZ may also be added in a stoichiometric ratio, such that the incompletely dissociated conjugated anion [Zⁿ⁻] is not formed but instead the respective protonated anions [HₘZ^{(n-m)-}] m < n are formed.

6. The method according to any one of Claims 1 to 5,
**characterized in that**
the reaction is performed in an aqueous, aqueous-organic or organic solvent (addition of polar solvents such as lower alcohols, ketones, sulfoxides, ethers, nitriles) or - in the case when the cationic synthesis module [Q⁺]ₙ[Yⁿ⁻] is liquid - is also performed without solvent.

7. The method according to any one of Claims 1 to 6,
**characterized in that**
the acid/base reaction is accelerated by applying a vacuum.

8. The method according to Claim 1 or 2,
**characterized in that**
in a type of reaction known as "metathesis," a suitable metal salt [M²⁺][Zⁿ⁻]_{2/n} as the anionic synthesis module, preferably a strontium, barium, zinc, manganese or calcium salt, is reacted with the carbonate precursor [Q⁺]₂[CO₃²⁻] as the cationic synthesis module, wherein the sparingly soluble metal carbonate [M²⁺][CO₃²⁻] is precipitated, so that the carbonate ion of the carbonate precursor [Q⁺]₂[CO₃²⁻] is replaced by the desired anion [Zⁿ⁻].

9. The method according to Claims 1, 2 or 8,
**characterized in that**
the precipitated sparingly soluble metal carbonate [M²⁺][CO₃²⁻] is separated from the desired ionic compound [Q⁺]ₙ[Zⁿ⁻] by filtration, centrifugation or other suitable methods.

10. The method according to any one of Claims 1, 2, 8 or 9,
**characterized in that**
the reaction is carried out in an aqueous, aqueous-organic or organic solvent (addition of polar solvents such as low alcohols, ketones, sulfoxides, ethers, nitriles] or - in the case when the cationic synthesis module Q⁺]₂[CO₃²⁻] and the desired ionic compound [Q⁺]ₙ[Zⁿ⁻] are liquid - is also performed as a solvent-free process.

11. The method according to any one of Claims 3 to 7,
**characterized in that**
the reaction is performed continuously or as a batch process or a semibatch process.

12. The method according to any one of Claims 1 to 10,
**characterized in that**
the reaction mixture is stirred, shaken or otherwise mixed.

13. The method according to any one of Claims 1 to 12,
**characterized in that**
after the reaction is concluded the solvent is removed by distillation, vacuum distillation, thin-film evaporation, rotary evaporation and other suitable methods.

14. The method according to any one of Claims 1 to 13,
**characterized in that**
conventional commercial industrial chemicals are used.

15. The method according to any one of Claims 1 to 12,
**characterized in that**
aqueous systems are used as the solvent.

16. The method according to any one of Claims 1 to 15,
**characterized in that**
the storable cationic synthesis modules are not reacted with the anionic synthesis modules until immediately before production of the ionic compound.

17. The method according to any one of Claims 1 to 16,
**characterized in that**
no halogen and/or silver compounds are used.

18. The method according to any one of Claims 1 to 17,
**characterized in that**
the ionic carbonate precursor [Q⁺]ₙ[Yⁿ⁻] (cationic synthesis module) is created by quaternization of linear or cyclic amines in NR¹,R²,R³, phosphines PR¹,R²,R³, sulfides SR¹R² or similar nitrogen, phosphorus, sulfur heteroaromatics in a nucleophilic substitution reaction with organic carbonic acid esters R⁴O(CO)OR⁵, where the radicals R¹,R²,R³,R⁴,R⁵ are all the same or may be partially the same or different, and these radicals are linear, cyclic, branched, saturated or unsaturated alkyl radicals, monocyclic or polycyclic aromatic or heteroaromatic radicals or derivatives of these radicals substituted with additional functional groups, wherein R¹,R²,R³ and R⁴,R⁵ may also be interconnected (multibond radical).

19. The method according to any one of Claims 1 to 18,
**characterized in that**
the ionic carbonate precursor [Q⁺]ₙ[Yⁿ⁻] (cationic synthesis module) is created by "metathesis" from an ionic precursor [Q⁺]ₙ[Xⁿ⁻] wherein a bicarbonate [Mⁿ⁺][HCO³]ₙ, a carbonate [Mⁿ⁺]₂[CO₃²⁻]ₙ or a monoalkyl and/or monoaryl carbonate [Mⁿ⁺] [RCO³]ₙ is added in a suitable dry solvent in the absence of atmospheric humidity, wherein [Mⁿ⁺] is a suitable metal cation and R is a linear, cyclic, branched, saturated or unsaturated alkyl radical, a monocyclic or polycyclic aromatic or heteroaromatic radical or a derivative of these radicals substituted with additional functional groups, wherein the desired ionic compounds [Q⁺]ₙ[Yⁿ⁻] (cationic synthesis module) is formed while stirring, the corresponding alkali or alkaline earth salt [Mⁿ⁺][X⁻] is precipitated as a solid and is removed by filtration in particular.

20. The method according to any one of Claims 1 to 17 or 19,
**characterized in that**
[Yⁿ⁻] is a bicarbonate [HCO₃⁻], [Xⁿ⁻] is a sulfate [SO₄²⁻] and [Mⁿ⁺] is a strontium or barium, especially preferably a calcium cation, wherein the quaternary ammonium, phosphonium, sulfonium or similar heteroaromatic sulfate [Q]₂⁺[SO₄²⁻] was created by alkylation or arylation of linear or cyclic amines NR¹,R²,R³, phosphines PR¹,R²,R³, sulfides SR¹R² or similar nitrogen, phosphorus, sulfur heteroaromatics by means of organic sulfates R⁴O(SO₂)OR⁵ and wherein the metal salt added to the sulfate [Q]₂⁺[SO₄²⁻] is a bicarbonate [Mⁿ⁺][HCO₃⁻]₂, which has first been created previously or *in situ* in aqueous or aqueous organic solution by reaction of the corresponding sparingly soluble alkaline earth carbonate [Mⁿ⁺][CO₃²⁻] by means of carbon dioxide, wherein the sparingly soluble strontium, barium or calcium sulfate precipitate is removed by filtration and the radicals R¹,R²,R³,R⁴,R⁵ are all the same or partially different or the same and wherein these radicals are linear, cyclic, branched, saturated or unsaturated alkyl radicals, monocyclic or polycyclic aromatic or heteroaromatic radicals or derivatives of these radicals substituted with additional functional groups, wherein R¹,R²,R³ and R⁴,R⁵ may also be interconnected (multibond radical).

21. The method according to any one Claims 1 to 17,
**characterized in that**
the ionic carbonate precursor [Q⁺]ₙ[Yⁿ⁻] (cationic synthesis module) is synthesized by a reaction of quaternary hydoroxides [Q⁺][OH⁻] and alcoholates [Q⁺][RO⁻] with carbon dioxide, where R is a linear, cyclic, branched, saturated or unsaturated alkyl radical, a monocyclic or polycyclic aromatic or heteroaromatic radical or a derivative of this radical substituted with additional functional groups.

22. The method according to any one Claims 1 to 17,
**characterized in that**
the ionic carbonate precursor [Q⁺]_{n[}Yⁿ⁻] (cationic synthesis module) by liquid ion exchange between a medium- to long-chain quaternary compound [Q'⁺]ₙ[Yⁿ⁻] and a quaternary compound [Q⁺]ₙ[Xⁿ⁻] in aqueous or aqueous-organic solution according to
[Q'⁺]ₙ[Yⁿ⁻] + [Q⁺]ₙ[Xⁿ⁻] → [Q⁺]ₙ[Yⁿ⁻] + [Q'⁺]ₙ[Xⁿ⁻]
wherein
[Q'⁺] is a quaternary ammonium cation [R'¹R'²R'³R'⁴N⁺], phosphonium cation [R'¹R'²R'³R'⁴P⁺] or sulfonium cation [R'¹R'²R'³S⁺] or a similar quaternized nitrogen, phosphorus or sulfur heteroaromatic,
wherein the radicals R'¹,R'²,R'³,R'⁴ are all the same or partially the same or different,
wherein the radicals R'¹,R'²,R'³ and R'⁴ are linear, cyclic, branched, saturated or unsaturated alkyl radicals, monocyclic or polycyclic aromatic or heteroaromatic radicals or derivatives of these radicals substituted with additional functional groups and
wherein R'¹,R'²,R'³,R'⁴ may also be interconnected (multibond radical),
wherein at least one of the radicals R'¹,R'²,R'³,R'⁴ has 4-30 carbon atoms,
wherein [Yⁿ⁻] is a bicarbonate [HCO₃], a carbonate [CO₃²⁻], a monoalkyl carbonate or a monoaryl carbonate [R'OCO₂⁻], and
wherein R' is a linear, cyclic, branched, saturated or unsaturated alkyl radical, monocyclic or polycyclic aromatic or heteroaromatic or [R'OCO₂⁻] is another monosubstituted carbonate,
wherein [Q⁺] is the desired cation of the ionic precursor,
wherein [Xⁿ⁻] is a chloride, bromide, iodide, sulfate, phosphate, p-toluene sulfonate, methane sulfonate, trifluoromethane sulfonate, trifluoroacetate anion or any other anion which is derived from a traditional quaternization reaction of a corresponding linear or cyclic amine NR¹R²R³, phosphine PR¹R²R³, sulfide SR¹R² or a similar nitrogen, phosphorus or sulfur heteroaromatic,
wherein [Q'⁺]ₙ[Xⁿ⁻] is separated as a non-water-miscible phase after successful liquid ion exchange,
wherein the desired ionic precursor [Q⁺]ₙ[Yⁿ⁻] is present in an aqueous or aqueous organic solution after separation of the non-water-miscible phase [Q'⁺]ₙ[Xⁿ⁻],
and wherein this precursor [Q⁺]ₙ[Yⁿ⁻] can finally be obtained by removing the solvent (evaporation, distillation).

## Revendications

1. Procédé de préparation de liquides ioniques, de matières solides ioniques ou de leurs mélanges à partir de modules de synthèse cationiques [Q⁺]ₙ[Yⁿ⁻] et de modules de synthèse anioniques,
[Q⁺] étant un cation quaternisé d'ammonium [R¹R²R³R⁴N⁺], de phosphonium [R¹R²R³R⁴P⁺] ou de sulfonium [R¹R²R³S⁺] ou un hétérocycle aromatique quaternisé analogue renferment de l'azote, du phosphore ou du soufre,
les radicaux R¹,R²,R³,R⁴ étant tous identiques, en partie identiques ou différents,
les radicaux R¹,R²,R³ et R⁴ étant des radicaux alkyle saturés ou insaturés de nature linéaire, cyclique, ramifiée, des radicaux aromatiques ou des hétérocycles aromatiques de nature mono- ou polycyclique, ou des dérivés desdits radicaux substitués avec d'autres groupes fonctionnels, et
R¹,R²,R³,R⁴ pouvant également être reliés entre eux (radical polyvalent),
[Yⁿ⁻] étant un bicarbonate [HCO₃], un carbonate [CO₃²⁻], un monoalkylcarbonate ou un monoarylcarbonate [ROCO₂⁻] et
R étant un radical alkyle saturé ou insaturé de nature linéaire, cyclique, ramifié, un composé aromatique ou un hétérocycle aromatique de nature mono- ou polycyclique, ou [ROCO₂⁻] étant un autre carbonate monosubstitué.

2. Procédé selon la revendication 1, **caractérisé en ce que** l'on fait réagir, de préférence, des modules de synthèse cationiques aptes au stockage avec des modules de synthèse anioniques en mettant en oeuvre un processus de production modulaire visant à obtenir des composés ioniques.

3. Procédé selon la revendication 1 ou 2, **caractérisé en ce que** l'on ajoute, dans une réaction acide-base selon Brønstedt, un acide de Brønstedt HₙZ à n bases servant de module de synthèse anionique au module de synthèse cationique [Q⁺]ₙ[Yⁿ⁻], faisant en sorte que l'anion de carbonate, de bicarbonate, de monoalkyl- ou monoarylcarbonate [Yⁿ⁻] s'échappe sous forme de dioxyde de carbone gazeux pour être remplacé par l'anion souhaité [Zⁿ⁻], les sous-produits générés étant de l'eau et, dans le cas des monoalkyl- ou monoarylcarbonates [ROCO₂⁻], les alcools ROH correspondants.

4. Procédé selon l'une des revendications 1 à 3, **caractérisé en ce que** l'ajout dudit acide de Brønstedt HₙZ à n bases est réalisé en masse ou après dissolution dans de l'eau ou dans d'autres solvants, de façon stoechiométrique ou excédentaire.

5. Procédé selon l'une des revendications 1 à 4, **caractérisé en ce que** l'ajout dudit acide de Brønstedt HₙZ à n bases peut également être réalisé selon une stoechiométrie telle que l'on n'obtient pas l'anion conjugué [Zⁿ⁻] sous sa forme complètement dissociée mais les anions protonés correspondants [HₘZ^{(n-m)-}], m < n.

6. Procédé selon l'une des revendications 1 à 5, **caractérisé en ce que** ladite réaction est réalisée dans un solvant aqueux, aqueux/organique, organique (ajout de solvants polaires tels que les alcools, cétones, sulfoxydes, éthers, nitriles à courte chaîne) ou bien - dans le cas d'un module de synthèse [Q⁺]ₙ[Yⁿ⁻] liquide - sans ajout de solvant.

7. Procédé selon l'une des revendications 1 à 6, **caractérisé en ce que** la réaction acide-base est accélérée par l'application d'un vide.

8. Procédé selon les revendications 1 ou 2, **caractérisé en ce que** l'on fait réagir, dans un type de réaction désigné par le terme « métathèse », un sel métallique approprié [M²⁺]Zⁿ⁻]_{2/n} servant de module de synthèse anionique, s'agissant préférentiellement d'un sel de strontium, de baryum, de zinc, de manganèse ou de calcium, avec un précurseur de carbonate [Q⁺]₂[CO₃²⁻] servant de module de synthèse cationique, ce qui provoque la précipitation de carbonate métallique peu soluble [M²⁺][CO₃²⁻], l'ion carbonate du précurseur de carbonate [Q⁺]₂[CO₃²⁻] étant ainsi remplacé par l'anion souhaité [Zⁿ⁻].

9. Procédé selon la revendication 1, 2 ou 8, **caractérisé en ce que** le carbonate métallique peu soluble [M²⁺][CO₃²⁻] précipité est séparé du composé ionique [Q⁺]ₙ[Zⁿ⁻] souhaité par filtration, centrifugation ou d'autres procédés appropriés.

10. Procédé selon l'une des revendications 1, 2, 8 ou 9, **caractérisé en ce que** ladite réaction est réalisée dans un solvant aqueux, aqueux/organique, organique (ajout de solvants polaires tels que les alcools, cétones, sulfoxydes, éthers, nitriles à courte chaîne) ou bien - au cas où le module de synthèse [Q⁺]₂[CO₃²⁻] cationique et le composé ionique [Q⁺]ₙ[Zⁿ⁻] souhaité seraient liquides - sans ajout de solvant.

11. Procédé selon l'une des revendications 3 à 7, **caractérisé en ce que** ladite réaction est mise en oeuvre de façon discontinue, semi-continue ou continue.

12. Procédé selon l'une des revendications 1 à 10, **caractérisé en ce que** le mélange réactionnel est agité par un mouvement circulaire ou de va-et-vient ou qu'il est mélangé autrement.

13. Procédé selon l'une des revendications 1 à 12, **caractérisé en ce qu'**une fois la réaction terminée, on élimine le solvant par distillation, distillation sous vide, évaporation en couche mince, évaporation rotative ou d'autres procédés appropriés.

14. Procédé selon l'une des revendications 1 à 13, **caractérisé en ce que** l'on met en oeuvre des produits chimiques de qualité industrielle qui sont disponibles dans le commerce.

15. Procédé selon l'une des revendications 1 à 14, **caractérisé en ce que** l'on utilise en tant que solvant des systèmes aqueux.

16. Procédé selon l'une des revendications 1 à 15, **caractérisé en ce que** lesdites modules de synthèse cationiques aptes au stockage ne sont mis en réaction avec lesdits modules de synthèse anioniques qu'immédiatement avant la production du composé ionique.

17. Procédé selon l'une des revendications 1 à 16, **caractérisé en ce que** l'on n'utilise pas de composés halogénés et/ou renfermant de l'argent.

18. Procédé selon l'une des revendications 1 à 17, **caractérisé en ce que** ledit précurseur de carbonate ionique [Q⁺]ₙ[Yⁿ⁻] (module de synthèse cationique) est obtenu par quaternisation d'amines NR¹R²R³, de phosphines PR¹R²R³, de sulfures SR¹R² de nature linéaire ou cyclique, ou d'hétérocycles aromatiques analogues renfermant de l'azote, du phosphore, du soufre, dans une réaction de substitution nucléophile mettant en oeuvre des esters d'acide carbonique R⁴O(CO)OR⁵, les radicaux R¹,R²,R³,R⁴,R⁵ étant tous identiques, en partie identiques ou différents et lesdits radicaux étant des radicaux alkyle saturés ou insaturés de nature linéaire, cyclique, ramifiée, des radicaux aromatiques ou des hétérocycles aromatiques de nature mono- ou polycyclique, ou des dérivés desdits radicaux substitués avec d'autres groupes fonctionnels, R¹,R²,R³ et R⁴,R⁵ pouvant être également reliés, respectivement, entre eux (radical polyvalent).

19. Procédé selon l'une des revendications 1 à 18, **caractérisé en ce que** ledit précurseur de carbonate ionique [Q⁺]ₙ[Yⁿ⁻] (module de synthèse cationique) est obtenu par « métathèse » à partir d'un précurseur [Q⁺]ₙ[Xⁿ⁻] qui est déjà ionique, en ajoutant un bicarbonate [Mⁿ⁺][HCO₃⁻]ₙ un carbonate [Mⁿ⁺][CO₃²⁻]ₙ ou un monoalkyl- ou monoarylcarbonate [Mⁿ⁺][RCO₃⁻]ₙ se trouvant dans un solvant anhydre approprié en éliminant toute humidité de l'air, [Mⁿ⁺] étant un cation métallique approprié et R étant un radical alkyle saturé ou insaturé de nature linéaire, cyclique, ramifiée, un radical aromatique ou un hétérocycle aromatique de nature mono- ou polycyclique, ou un dérivé desdits radicaux substitué avec d'autres groupes fonctionnels, le composé ionique [Q⁺]ₙ[Yⁿ⁻] souhaité (module de synthèse cationique) étant formé sous agitation, le sel alcalin ou alcalino-terreux [Mⁿ⁺][X⁻] correspondant étant précipité sous forme solide et éliminé notamment par filtration.

20. Procédé selon l'une des revendications 1 à 17 ou 19, **caractérisé en ce que** [Yⁿ⁻] est un bicarbonate [HCO₃⁻], que [Xⁿ⁻] est un sulfate [SO₄²⁻] et que [Mⁿ⁺] est un strontium ou baryum, avec une préférence particulière un cation de calcium, le sulfate d'ammonium, de phosphonium, de sulfonium quaternaires ou d'un hétérocycle aromatique analogue [Q]₂⁺[SO₄²⁻] étant obtenu par alkylation ou arylation, au moyen de sulfates organiques R⁴O(SO₂)OR⁵, d'amines NR¹R²R³, de phosphines NR¹R²R³, de sulfures SR¹R² de nature linéaire ou cyclique ou d'hétérocycles aromatiques analogues renfermant de l'azote, du phosphore, du soufre, et le sel métallique ajouté audit sulfate (Q)₂⁺[SO₄²⁻] étant un bicarbonate [Mⁿ⁺][HCO₃⁻]₂ qui a été obtenu préalablement ou in situ en faisant réagir, dans une solution aqueuse ou aqueuse-organique, le carbonate alcalino-terreux peu soluble [Mⁿ⁺][HCO₃⁻]₂ correspondant en mettant en oeuvre du dioxyde de carbone, le sulfate peu soluble de strontium, de baryum ou de sulfate ainsi précipité étant éliminé notamment par filtration, et les radicaux R¹,R²,R³,R⁴,R⁵ étant aussi tous identiques, en partie identiques et différents, et lesdits radicaux étant des radicaux alkyle saturés ou insaturés de nature linéaire, cyclique, ramifiée, des radicaux aromatiques ou des hétérocycles aromatiques de nature mono- ou polycyclique, ou des dérivés desdits radicaux substitués avec d'autres groupes fonctionnels, R¹,R²,R³ et R⁴,R⁵ pouvant être également reliés, respectivement, entre eux (radical polyvalent).

21. Procédé selon l'une des revendications 1 à 17, **caractérisé en ce que** ledit précurseur de carbonate ionique [Q⁺]ₙ[Yⁿ⁻] (module de synthèse cationique) est obtenu en faisant réagir des hydroxydes quaternaires [Q⁺][OH⁻] et des alcoolates [Q⁺][RO⁻] avec du dioxyde de carbone, R étant un radical alkyle saturé ou insaturé de nature linéaire, cyclique, ramifiée, un radical aromatique ou un hétérocycle aromatique de nature mono- ou polycyclique, ou un dérivé desdits radicaux substitué avec d'autres groupes fonctionnels.

22. Procédé selon l'une des revendications 1 à 17, **caractérisé en ce que** ledit précurseur de carbonate ionique [Q⁺]ₙ[Yⁿ⁻] (module de synthèse cationique) résulte d'un échange d'ions en milieu liquide entre un composé quaternaire à chaîne moyenne à longue [Q⁺]ₙ[Yⁿ⁻] et un composé quaternaire [Q⁺]ₙ[Xⁿ⁻] dans une solution aqueuse ou aqueuse-organique selon
[Q'⁺]ₙ[Yⁿ⁻] + [Q⁺]ₙ[Xⁿ⁻] → [Q⁺]ₙ[Yⁿ⁻] + [Q'⁺]ₙ[Xⁿ⁻]
[Q'⁺] étant un cation quaternisé d'ammonium [R'¹R'²R'³R'⁴N⁺], de phosphonium [R'¹R'²R'³R'⁴P⁺] ou de sulfonium [R'¹R'²R'³S⁺] ou un hétérocycle aromatique quaternisé analogue renfermant de l'azote, du phosphore ou du soufre,
les radicaux R'¹R'²R'³R'⁴ étant tous identiques, partiellement identiques ou différents,
les radicaux R'¹, R'², R'³ et R'⁴ étant des radicaux alkyle saturés ou insaturés de nature linéaire, cyclique, ramifiés, des radicaux aromatique ou des hétérocycles aromatiques de nature mono- ou polycyclique, ou des dérivés desdits radicaux substitués avec d'autres groupes fonctionnels, et
R'¹,R'²,R'³,R'⁴ pouvant également être reliés entre eux (radical polyvalent),
au moins un des radicaux R'¹,R'²,R'³,R'⁴ comportant 4 à 30 atomes de carbone, [Yⁿ⁻] étant un bicarbonate [HCO₃⁻], un carbonate [CO₃²⁻
], un monoalkylcarbonate ou un monoarylcarbonate [R'OCO₂⁻] et
R' étant un radical alkyle saturé ou insaturé de nature linéaire, cyclique, ramifié, un composé aromatique ou un hétérocycle aromatique de nature mono- ou polycyclique, ou [R'OCO₂⁻] étant un autre carbonate monosubstitué,
[Q⁺] étant le cation souhaité dudit précurseur ionique,
[Xⁿ⁻] étant un anion chlorure, bromure, iodure, sulfate, phosphate, p-toluènesulfonate, méthansulfonate, trifluorométhanesulfonate, trifluoroacétate ou tout autre anion résultant d'une réaction de quaternisation classique que subit une amine NR¹R²R³, phosphine NR¹R²R³, sulfure SR¹R² correspondant de nature linéaire ou cyclique ou un hétérocycle aromatique analogue renfermant de l'azote, du phosphore, du soufre.
[Q'⁺]ₙ[Xⁿ⁻] se séparant sous forme d'une phase non miscible à l'eau, une fois l'échange d'ions en milieu liquide terminé,
le précurseur ionique souhaité [Q⁺]ₙ[Yⁿ⁻] se trouvant dans une solution aqueuse ou aqueuse-organique, une fois la phase non miscible à l'eau [Q'⁺]ₙ[Xⁿ⁻] séparée,
et ce précurseur [Q⁺]n[Yⁿ⁻] pouvant enfin être obtenu par élimination du solvant (évaporation, distillation).
